# EUROPEAN PATENT APPLICATION

(11) **EP 2 008 710 A1**
(43) Date of publication of application: **31.12.2008**
(21) Application number: 07741430.8
(22) Date of filing: 11.04.2007
(51) Int. Cl.: B01J 13/04, A61K 9/127, A61K 47/24

(54) **PROCESS AND APPARATUS FOR PRODUCING LIPOSOME DISPERSION**

(30) Priority: 11.04.2006 JP 2006109094
(71) Applicant: Wingturf Co., Ltd., Tokyo 100-6510 (JP); Hashiba, Tomohiko, Tokyo 101-0021 (JP)
(72) Inventor: HASHIBA, Tomohiko, Chiyoda-ku, Tokyo 1006510 (JP)
(74) Representative: Claessen, Rolf
(86) International application number: PCT/JP2007/057994
(87) International publication number: WO 2007/117023

(57) **Abstract**

Provided are a production method of liposome dispersion industrially useful and the production equipment.

In a process for forming liposome dispersion by dispersing a membrane material in an aqueous medium, wherein a liquid that the membrane material is dissolved or dispersed is discharged from 161a, while discharging the aqueous medium from 161b, the discharge flows are collectively crushed by a high-speed gas stream from a liquid jet port 162 into minute droplets, then, the droplets are crashed against a flow-blocking body 190 to reaggregate for the production. According to the production of the present invention, since removal of solvent is easy, and there is no need of defoaming operation, it becomes possible to produce liposome dispersion efficiently.

## Description

### TECHNICAL FIELD

The present invention relates to a method for preparing liposome usable as drug carriers in medicine, foods and cosmetics.

### BACKGROUND ART

Liposome is in a lipid bilayer or multilayer composed mainly of phospholipids, having a similar structure to a cell membrane. A watersoluble drug is taken into an inner water phase of liposome, and a fat-soluble drug is taken into a lipid layer thereof. There have been studied applications of liposome as a drug delivery system in medicine and cosmetic fields, and proposed methods for preparing various kinds of liposome as well.

As the methods for preparing liposome that have been reported so far, there are listed the preparation methods: (1) lipid is dissolved in an organic solvent (chloroform or the like), the solvent is removed under reduced pressure to form a thin membrane, then, an aqueous solution such as buffer solution is added and stirred to swell, further the thin membrane is peeled by a mechanical stirring means; (2) lipid is dissolved in an organic solvent such as ether or ethanol, poured in an aqueous solution such as buffer solution, then, the solvent is removed; further as a mechanical method, (3) method by ultrasonication; (4) method by a high-pressure homogenizer and high-speed rotary dispersing machine (see Patent document 1); (5) method by high-pressure filtration using a membrane filter made of polycarbonate, and the like.

Further, a method for preparing liposome by a microcapsulation has been reported (see Non-patent document 1). In this method, lipid is dissolved in an organic solvent not mixable with water (hexane, methylene chloride or the like), after preparing a W/O type emulsion by adding water using a homogenizer, the emulsion is added to water, thereby to prepare liposome.

However, these methods have problems that they are not industrial ones because operations are tedious and solvents need to be distilled away.
[Patent document 1] Japanese Unexamined Patent Publication No. 11-139961 (1999)
[Non-patent document 1] J. Dispersion Sci. Technol., 9, 1 (1988)

### DISCLOSURE OF THE INVENTION

An object of the present invention to solve the problems is to provide a new production method of liposome dispersion industrially useful and the production equipment.

The present inventors keenly studied, as a result, they have found that by spraying a membrane material forming liposome and an aqueous medium to hydrate it using a two-fluid nozzle, with no need of defoaming treatment, liposome dispersion with a small particle diameter and a very narrow distribution thereof can be prepared. They have found that the dispersion state in spraying is very reactive, and also in using an organic solvent, the removal can be done very efficiently, and completed the present invention.

Namely, the present invention relates to a production method of liposome dispersion, in a process for forming liposome by dispersing a membrane material in an aqueous medium, wherein the membrane material is dispersed in the aqueous medium by spray mixing.

The present invention relates to the production method, wherein while discharging a liquid that the membrane material is suspended in the aqueous medium, the discharge flow is crushed by a gas stream to produce minute droplets, then, the droplets are aggregated.

The present invention relates to the production method, wherein while discharging a liquid that the membrane material is dissolved or dispersed in a solvent and the aqueous medium, the discharge flow is crushed by a gas stream to produce minute droplets, then, the droplets are aggregated.

The present invention further relates to production equipment of liposome dispersion, which is equipment for producing liposome dispersion, comprising; a nozzle equipped with: a first discharge port for discharging a liquid that a membrane material is dissolved or dispersed, a second discharge port equipped adjacent to the first discharge port for discharging an aqueous medium, and a gas jet port jetting a gas for collectively crushing discharge flows from the first discharge port and the second discharge port to produce minute droplets; a first solution-sending means for supplying a liquid that the membrane material is dissolved or dispersed to the nozzle; a second solution-sending means for supplying the aqueous medium to the nozzle; and a gas supply means for supplying the gas to the nozzle.

The present invention further relates to the production equipment, wherein the first discharge port is circular, the second discharge port is formed in an annular shape surrounding the first discharge port, and the gas jet port is formed in the periphery of the second discharge port.

The present invention further relates to the production method, wherein a flow-blocking body is provided oppositely to the first discharge port and the second discharge port, and constituted so that a flow of minute droplets produced by crushing the liquid that the membrane material is dissolved or dispersed and the aqueous medium by the gas stream is aggregated by crashing against the flow-blocking body.

According to the production method of liposome dispersion and the production equipment of the present invention, it is possible to prepare liposome dispersion efficiently without conducting tedious operations.

### BRIEF DESCRIPTION OF THE DRAWINGS

Fig. 1 is a block diagram showing preferable production equipment 100 for conducting a production method of liposome dispersion according to the present invention;
Fig. 2 is a diagram explaining a two-fluid nozzle 160 of production equipment 100, Fig. 2(a) is the perspective view and Fig. 2(b) is the cross-sectional view;
Fig. 3 is a diagram explaining a casing 160A of production equipment 100, (a) is the perspective view and (b) is the cross-sectional view;
Fig. 4 is a diagram explaining a core 160B of production equipment 100, (a) is the perspective view and (b) is the cross-sectional view;
Fig. 5 is a diagram explaining a base 160C of production equipment 100, Fig. 5(a) is the perspective view and Fig. 5(b) is the cross-sectional view;
Fig. 6 is a diagram explaining a solution-sending tube 191 of production equipment 100;
Fig. 7 is a diagram explaining a tube holder 192 of production equipment 100, Fig. 7(a) is the perspective view, Fig. 7(b) is the plan view and Fig. 7(c) is the cross-sectional view;
Fig. 8 is a diagram explaining a connecting member 193 of production equipment 100, Fig. 8(a) is the perspective view, Fig. 8(b) is the plan view and Fig. 8(c) is the cross-sectional view;
Fig. 9 is an elevation for explaining a two-fluid nozzle 160 of production equipment 100; and
Fig. 10 is a block diagram showing a constitutional example of the control device of production equipment 100.

### DESCRIPTION OF REFERENCE NUMERALS

- 100: Production equipment
- 110: Raw material liquid supply system
- 111a,: 111b Raw material tank
- 112a,: 112b Raw material liquid
- 121a,: 121b Raw material feeding pipe
- 121i,: 121j Inlet
- 121o,: 121p Outlet
- 122i,: 122j Strainer
- 123a,: 123b, 134a, 134b, 137, 140 Electromagnetic valve
- 124: Dispersion
- 125: Recovery container
- 131a,: 131b Pressure piping
- 131o,: 131p Outlet
- 132: Branch pipe
- 133: Compressor
- 135a,: 135b, 138 Pressure sensor
- 136: Gas supply pipe
- 139: Compressed gas reservoir
- 151a,: 151b Liquid supply port
- 152: Gas supply port
- 160: Two-fluid nozzle
- 160A: Casing
- 160B: Core
- 160C: Base
- 161a,: 161b Liquid discharge port
- 162: Gas jet port
- 163: Contour
- 164a,: 164b Feeding pipe connection hole
- 165: Through-hole
- 165e: Diameter expansion part
- 165f: Catching part
- 166,: 173 Female screw groove
- 167,: 169 Step part
- 168: Flow channel hole
- 170: Space of approximate cylindrical shape
- 171,: 175, 178, 179 Male screw groove
- 172: O-ring
- 174: Protruding portion
- 176: Spiral forming body
- 177: Swirl chamber
- 179: Circling groove
- 180: Control device
- 182: MPU
- 181: ROM
- 183: RAM
- 184: Interface unit
- 185: A/D converter
- 186: Driving unit
- 187: Busline
- 188: Display device
- 189: Input device
- 190: Flow-blocking body (baffle board)
- 191: Solution sending tube
- 191h: Head part
- 192: Tube holder
- 192d: Body part
- 192h: Head part
- 193: Connection member
- 193r: Cylindrical part
- 193s: Seat part
- 194: Flow channel
- 196: Gap
- 197, 198: Nut

### BEST MODE FOR CARRYING OUT THE INVENTION

Hereinafter, the present invention is detailed along the best mode for carrying out the present invention.

### (Production method of liposome dispersion)

In the production method of liposome dispersion according to the present invention, as a membrane material for forming liposome, it is not particularly limited, but specifically, phospholipids, glycolipids and the like are listed. For example, phospholipids include soybean lecithin, hydrogenated soybean lecithin, egg-yolk lecithin, hydrogenated egg-yolk lecithin, phosphatidyl choline, phosphatidyl ethanolamine, phosphatidyl serine, alone or a mixture thereof. There can be used synthetic phospholipids such as dimyristoyl phosphatidyl choline, dipalmitoyl phosphatidyl choline, distearoyl phosphatidyl choline and dioleoyl phosphatidyl choline. Further, lysophospholipids can be used when it is used as a mixture. Glycolipids include cerebroside, globoside, ganglioside and the like. These lipids can also be used in the arbitral combination.

For prevention of oxidation and stabilization of liposome, lipids to which fatty acids such as tocophenol, cholesterol and palmitic acid are added can be used.

In the production method of liposome dispersion according to the present invention, as an aqueous medium to hydrate a membrane substance, it is not particularly limited, specifically, water, buffers such as phosphate buffer and citrate buffer, physiological saline and the like are listed.

In the production method of the present invention, various additives can be added for compounding in liposome dispersion. The additive is not particularly limited, and may suitably be selected by matching applications such as medicines and cosmetics and properly added to a raw material to be discharged. For example, there can be added anticancer drugs such as cytosine arabinoside, methotrexate and adriamycin; antibacterial agents such as amphotericin B, gentamysine and piperacillin; hepatic such as glutathione; enzymes such as superoxide dismutase and glucoamylase; biologically active substances such as tumor necrosis factor (TNF), epidermal growth factor (EGF) and erythropoietin; hormones such as prostaglandin and steroid; immunomodulators such as muramyl dipeptide, lymphokine and lenthinan; nucleic acids such as DNA and RNA; vitamins such as vitamin A and its derivatives, vitamin Bs and their derivatives, vitamin C and its derivatives, vitamin E and its derivatives, vitamin Ds, pantothenic acid, and nicotinic-acid amide, moisturizing agents such as hyaluronic acid, chondroitin sulfate, α-hydroxy acid, sorbitol, xylitol and maltitol, polyhydric alcohol such as propylene glycol, 1,3-butylene glycol and glycerin, fats and oils such as avogado oil, olive oil, mink oil, fatty acids, sphingolipid, lysophospholipid, squalene, squalane, and sterols and the derivatives, thickeners such as polyvinyl alcohol and methyl cellulose, organic solvents such as ethanol, other organic acids, pH modifier, antiseptic, antiphlogistic, chelating agent, natural extract and fragrance.

The production method of liposome dispersion according to the present invention is characterized in that in a process for forming liposome by dispersing a membrane material in an aqueous medium, the membrane material is dispersed in the aqueous medium by spray mixing.

Herein, spray mixing in the present invention means that a plurality of components are mixed by spraying in a state of misty particulates. A device used for spray mixing is not particularly limited, and a two-fluid nozzle generally known can be preferably used. More specifically, it can be referred to two-fluid nozzles described in Japanese Unexamined Patent Publications Nos. 2005-295856 and 2003-62493 and the like. A new nozzle equipped with two discharge ports described below may also be used.

A method for supplying a membrane material and an aqueous medium to a spray mixing means (in particular, a two-fluid nozzle generally known) is not particularly limited. For example, it can suspend a membrane material in an aqueous medium and supply the suspension directly to a two-fluid nozzle, or it may supply a mixture of a liquid that a membrane material is dissolved or dispersed in a solvent and an aqueous medium.

In regard to stirring when a membrane material is added to an aqueous medium and suspended, a high stirring is not required, and it is enough that a membrane material is dispersed as a powder in an aqueous medium ordinarily in stirring at about 200 to 1000 rpm.

As a solvent for a membrane material to be dissolved or dispersed, it is not particularly limited, there can be preferably exemplified organic solvents such as dimethyl ether, diethyl ether, n-hexane, ethanol, chloroform and n-decane, and their mixed solvent.

The production method of liposome dispersion according to the present invention can prepare liposome dispersion very efficiently.

For example, by suspending a membrane material as a powder in an aqueous medium and spraying, it is possible to prepare liposome with a uniform particle diameter without using an organic solvent. Further, even when an organic solvent is used, since removal of an organic solvent is easy in spraying as described above, it is possible to diminish or omit the following organic solvent removal treatment.

In a conventional dispersion method using a high-pressure homogenizer (Drug Development and Industrial Pharmacy, 16(14), 2167, (1990)), a high-speed rotary dispersing machine "Cleamix^{™}" or the like, there is a case that it needs to remove a gas dissolved in dispersion, but in the production method according to the present invention, there is very few residual bubble in the dispersion prepared.

### (Production equipment of liposome dispersion)

Hereinafter, preferable production equipment for conducting a production method of liposome dispersion according to the present invention will be detailed in reference to the drawings.

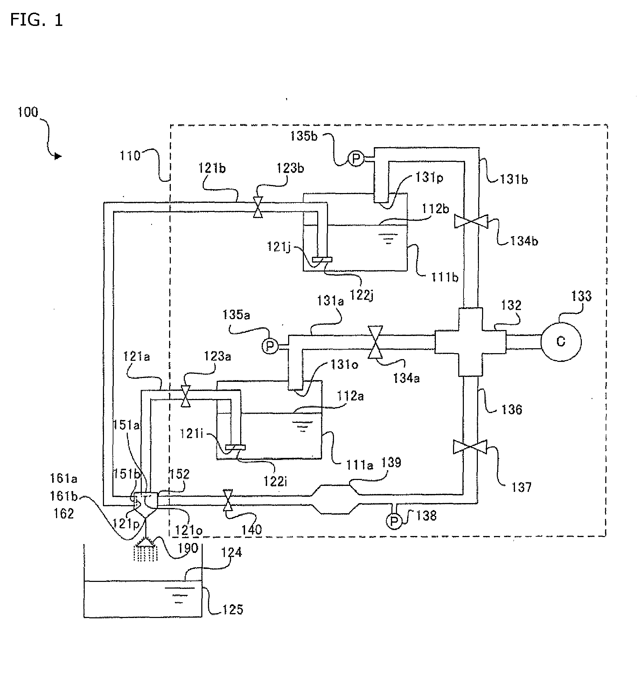

Fig. 1 is a block diagram showing preferable production equipment for conducting a production method of liposome dispersion according to the present invention.

Production equipment 100 is equipped with a raw material supply system 110, two-fluid nozzle 160 and flow-blocking body (baffle board) 190.

The raw material supply system 110 is equipped with two raw material tanks 111a and 111b. The raw material tanks 111a and 111b are each a sealable pressure container, and each sealed after raw material liquids 112a and 112b are poured.

To the first raw-material tank 111a, a raw material feeding pipe 121a is connected. An inlet 121i of raw material feeding pipe 121a is disposed in the vicinity of the inner bottom face of raw material tank 111a. To the inlet 121i of raw material feeding pipe 121a, a strainer 122i is attached. An outlet 121o of raw material feeding pipe 121a is connected to a liquid supply port 151a of two-fluid nozzle 160. In the intermediate part of raw material feeding pipe 121a, an electromagnetic variable throttle 123a for flow rate adjustment is provided.

To the second raw material tank 111b, a raw material feeding pipe 121b is connected. An inlet 121j of raw material feeding pipe 121b is disposed in the vicinity of the inner bottom face of raw material tank 111b. To the inlet 121j of raw material feeding pipe 121b, a strainer 122j is attached. An outlet 121p of raw material feeding pipe 121b is connected to a liquid supply port 151b of two-fluid nozzle 160. In the intermediate part of raw material feeding pipe 121b, an electromagnetic variable throttle 123b for flow rate adjustment is provided.

To the first raw-material tank 111a, a pressure piping 131a is connected through penetrating the ceiling wall thereof. The outlet 131o of pressure piping 131a is disposed in the vicinity of the ceiling wall of raw material tank 111a. The pressure piping 131a is a piping for introducing a compressed gas to an upper space inside the raw material tank 111a (space present above raw material liquid 112a).

To the second raw-material tank 111b, a pressure piping 131b is connected through penetrating the ceiling wall thereof. The outlet 131p of pressure piping 131b is disposed in the vicinity of the ceiling wall of raw material tank 111b. The pressure piping 131b is a piping for introducing a compressed gas to an upper space inside the raw material tank 111b (space present above raw material liquid 112b).

The edge of the uppermost stream of both pressure piping 131a and 131b is each connected to a compressed gas exit of compressor 133 via a branch pipe 132. In the way of pressure piping 131a, an electromagnetic valve 134a is provided, and also a pressure sensor 135a for detecting atmospheric pressure inside the upper space of raw material tank 111a is provided. In the way of pressure piping 131b, an electromagnetic valve 134b is provided, and also a pressure sensor 135b for detecting atmospheric pressure inside the upper space of raw material tank 111b is provided.

To a gas supply port 152 of two-fluid nozzle 160, a gas supply pipe 136 is connected. The edge of the uppermost stream of gas supply pipe 136 is connected to an exit of compressor 133 via a branch pipe 132. Namely, in the branch pipe 132, the outlet is branched into three, to which pressure piping 131a and 131b and a gas supply pipe 136 are each connected. In the way of gas supply pipe 136, in order from the upper stream side to the lower stream side, an electromagnetic valve 137, pressure sensor 138, compressed gas reservoir 139 and pressure control valve 140 are provided. The pressure sensor 138 is a sensor for detecting atmospheric pressure inside the compressed gas reservoir 139.

The compressor 133 is one for generating a compressed gas. The compressed gas discharged from the compressor 133 is distributed through the branch pipe 132 into pressure piping 131a and 131b and a gas supply pipe 136. The gas supply pipe 136 is a piping for introducing a compressed gas to a two-fluid nozzle 160. The compressed gas supplied to the gas supply pipe 136 is stored in a compressed gas reservoir 139, and introduced to the two-fluid nozzle 160 after being adjusted to a predetermined pressure.

At the edge part of two-fluid nozzle 160, a liquid discharge port 161a, a liquid discharge port 161b and a gas jet port 162 are provided. The gas jet port 162 is formed in the periphery of liquid discharge port 161a and 161b.

Around the lower side of two-fluid nozzle 160, a flow-blocking body 190 made of stainless steel is provided. The flow-blocking body 190 is a conical member with an upwardly reduced diameter, the tip (top edge) is facing both liquid discharge ports 161a and 161b of two-fluid nozzle 160. The two-fluid nozzle 160 and flow-blocking body 190 are both accommodated in a right cylindrical body not shown in the figure, and held in juncture of the inner wall of the right cylindrical body.

A raw material liquid 112a supplied to the liquid supply port 151a of two-fluid nozzle 160 is discharged from a liquid discharge port 161a, and a raw material liquid 112b supplied to the liquid supply port 151b is discharged from a liquid discharge port 161b, and in front of two-fluid nozzle 160 (downward in the figure), a high-speed swirling stream of air jetted from a gas jet port 162 is formed, and the discharged raw material liquids 112a and 112b are crushed into minute particles (misty) by this high-speed swirling stream. Then, the flow right after being crushed crashes against the flow-blocking body 190. As a result, the crushed flow reaggregates (reaggregation of misty particulates each other) right after being crushed, to produce a dispersion 124 in a state that two raw material liquids became uniform. By reaggregation on the flow-blocking body 190, the dispersion 124 flows down along the surface of flow-blocking body 190, and the dispersion 124 fallen from the bottom edge of flow-blocking body 190 gathers in a product container 125.

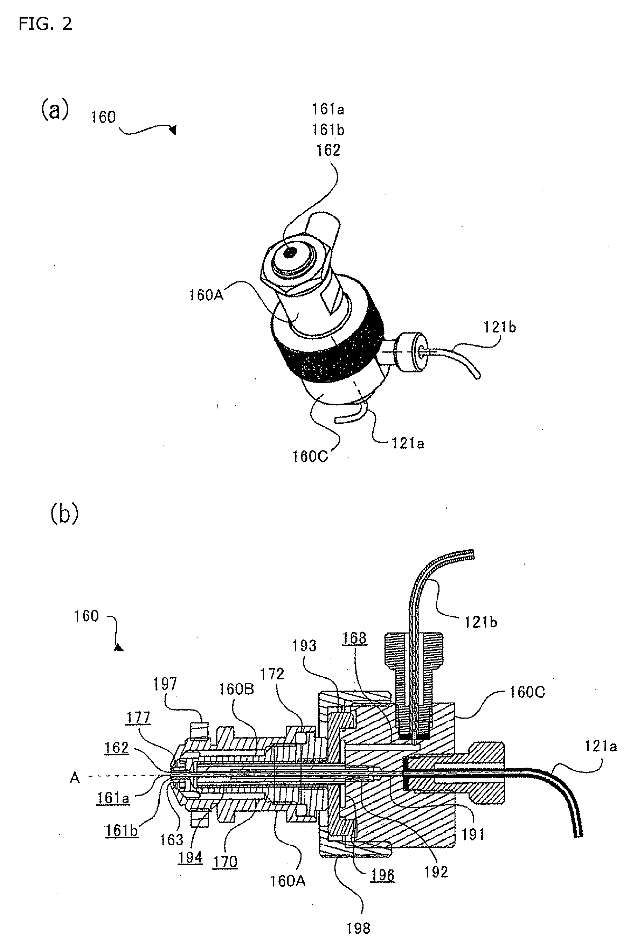

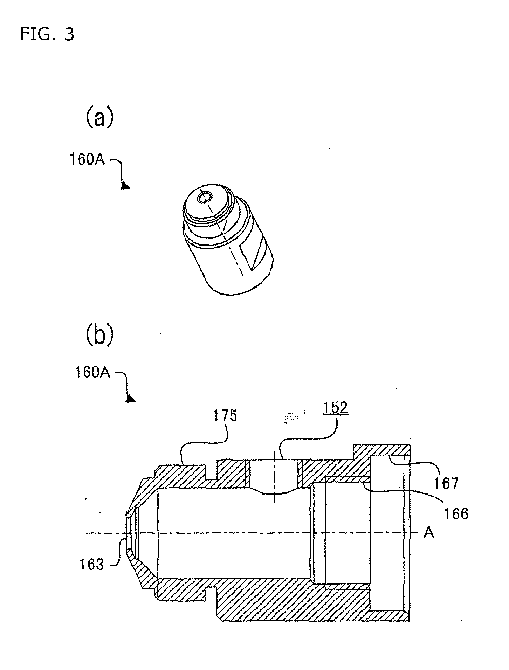

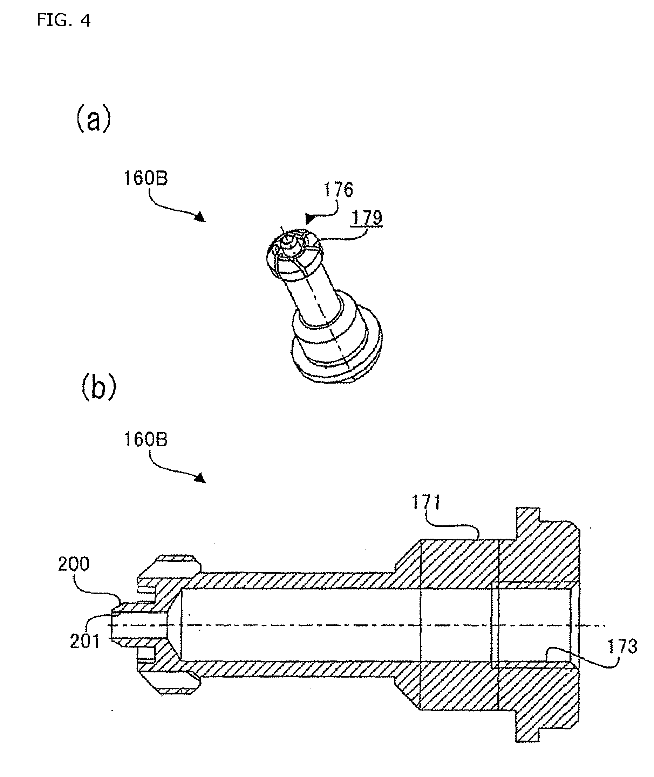

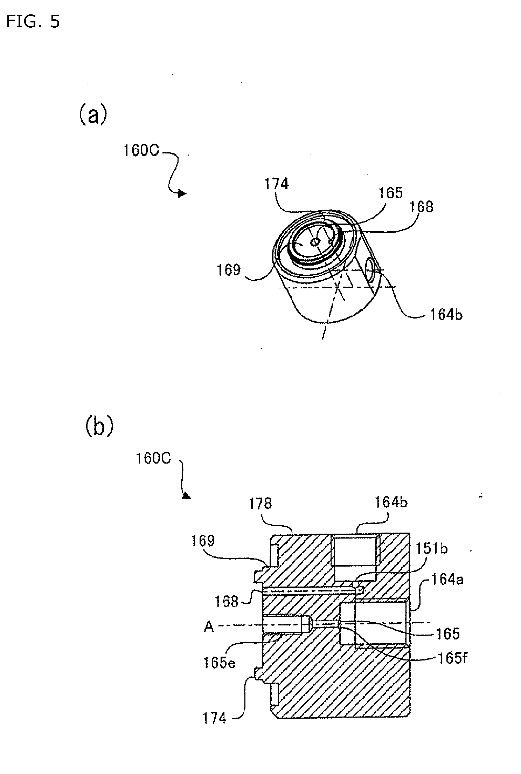

Next, the structure of two-fluid nozzle 160 is explained in reference to Figs. 2 to 9.

The two-fluid nozzle 160 is equipped with an approximately cylindrical hollow casing 160A , an approximately cylindrical core 160B inserted and screwed inside the casing 160A, and a base 160C to which raw material feeding pipes 121a and 121b are connected.

The casing 160A is a member produced by machining a metal material such as stainless steel and brass. At the tip of casing 160A, a circular opening 163 is formed. The center of opening 163 falls in with a central axial line A of two-fluid nozzle 160 at the center. The tip fringe of this opening 163 forms a contour of gas jet port 162. In the side face of casing 160A, a gas supply port 152 is bored. Around the inner periphery of gas supply port 152, a female screw groove is cut, into which a gas supply pipe 136 is threaded and joined. At the base edge side of the inner face of casing 160A, a female screw groove 166 is formed, and further at the base edge side, a step part 167 with a somewhat wider inner diameter is formed. On the outer surface near the tip of casing 160A, a female screw groove 175 is formed, by which a nut 197 for attaching the two-fluid nozzle 160 can be fixed.

A core 160B is produced by machining the same metal material as or different from the foregoing casing 160A, and the inside is bored along the central axis line A into hollow. The outer diameter size of a straight part of core 160B is chosen to be somewhat smaller than the inner diameter size of casing 160A, thereby to form a cylindrical space 170 to the inner surface of casing 160A. This space 170 communicates with a gas supply port 152 provided in the casing 160A. Around the periphery somewhat at the tip side from the base edge of core 160B, a male screw groove 171 is cut, which is screwed together with the foregoing female screw groove 166, and the core 160B is fixed inside the casing 160A. A part at the base edge side further from the male screw groove 171 is somewhat larger diameter, and an O-ring seal 172 is held between this part and the foregoing step part 167 to ensure the gas-tightness of the foregoing space 170. At base edge in the inner surface of core 160B, a female screw groove 173 is formed.

A base 160C is an approximately cylindrical member. In the side face of base 160C, a feeding pipe connection hole 164b is formed, and in the edge face of the base edge side, a feeding pipe connection hole 164a is formed. In the bottom face of feeding pipe connection hole 164b, a liquid supply port 151b is formed. Around the inner periphery of feeding pipe connection hole 164a, a female screw groove is cut, into which the tip of liquid feeding pipe 121a is threaded and joined. Around the inner periphery of feeding pipe connection hole 164b, a female screw groove is cut, into which the tip of liquid feeding pipe 121b is threaded and joined. In the bottom face of feeding pipe connection hole 164a, a through-hole 165 penetrating the base 160C is formed. The through-hole 165 has a diameter expansion part 165e formed by expanding a diameter from the intermediate part. The edge part of base edge side of through-hole 165 has a catching part 165f formed by expanding a diameter. At the tip side in the side face of base 160C, a male screw groove 178 is formed. In the edge face of the tip side of base 160C, a step part 169 formed by reducing a diameter is formed. In the step part 169, a flow channel hole 168 being the flow channel of raw material liquid 121b communicating with the liquid supply port 151b is bored. In the step part 169, a cylindrical protruding portion 174 outside a diameter direction further from the flow channel hole 168 is formed.

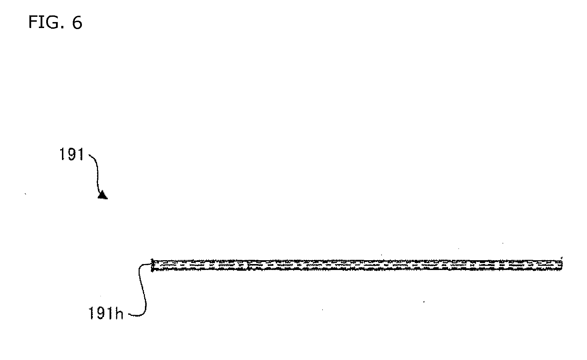

Into the through-hole 165, a solution sending tube 191 is inserted from the base edge side. As shown in Fig. 6, the solution sending tube 191 has a head part 191h that a diameter is expanded at its one end. The head part 191h is engaged with the foregoing catching part 165f. On the edge face of this head part 191h, the edge face of liquid feeding 121a is contacted and joined. The opening of the base edge side of this solution sending tube 191 constitutes a liquid supply port 151a, and the opening of the tip side constitutes a liquid discharge port 162a.

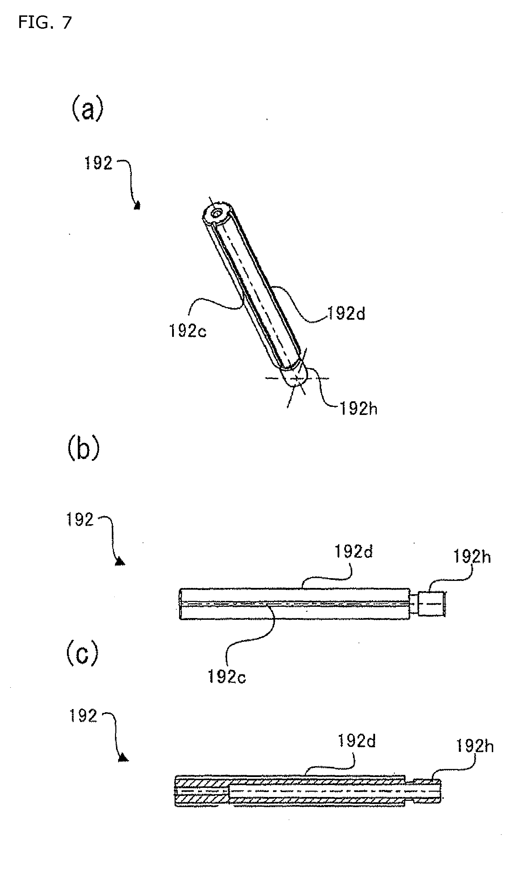

A tube holder 192 is fit in the through-hole 165 from the tip side. As shown in Fig. 7, the tube holder 192 is an approximately cylindrical member, and has a body part 192d and a head part 192h. In the tube holder 192, the foregoing solution sending tube 191 is penetrating the inside. The head part 192h of tube holder 192 is fixed in the foregoing diameter expansion part 165e. In the body part 192d of tube holder 192, four grooves 192c extending toward the longitudinal direction are formed in a circumferential direction.

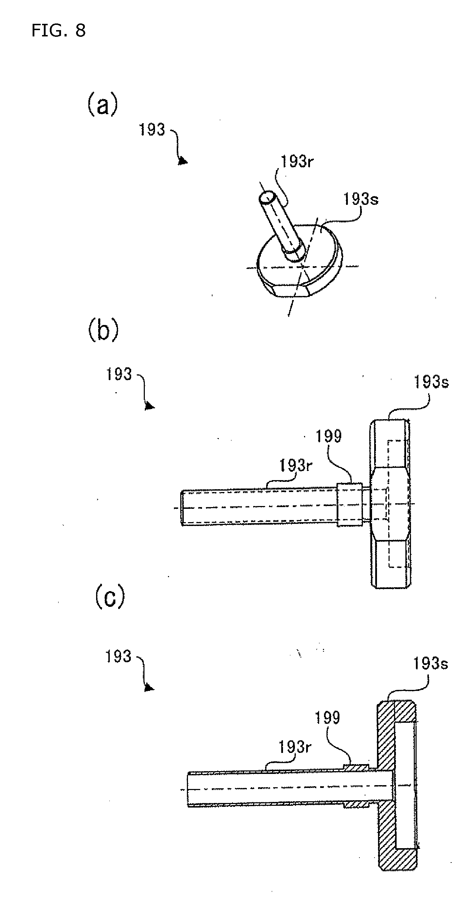

The core 160B and base 160C are joined by a connection member 193 and a fixed nut 198. As shown in Fig. 8, the connection member 193 is equipped with a seat part 193s and a cylindrical part 193r standing on the seat part 193s. The inner diameter size of cylindrical part 193r is set so that the body part 192d of tube holder 192 is precisely fit therein, and when the tube holder 192 is accommodated, a space between the inner face of cylindrical part 193r and the outer face of tube holder 192 is made by the foregoing groove 192c, thereby to form a flow channel 194. The outer diameter size of cylindrical part 193r is set so that it is precisely fit in the hollow hole of core 160B. At the crotch part of cylindrical part 193r, a male screw groove 199 is formed, with which the foregoing female screw groove 173 of the core 160B is screwed together, and joined with the core 160B. On the inner face of nut 198, a female screw groove to be screwed with a male screw groove 178 of base 160C is formed. The connection member 193 accommodates the body part 192d of tube holder 192, which is joined with the base 160C by a fixed nut 198 in a state that the step part 169 is fit in the inner face of seat part 193s. In this case, by the protruding portion 174, a flow channel hole 168 between the inner face of seat part 193s and step part 169, and a gap 196 communicating with the foregoing flow channel 194 are produced.

An expanded part of approximately conical shape at the edge part of core 160B forms a spiral forming body 176. A swirl chamber 177 is formed between the edge face of spiral forming body 176 and the inner face of the tip of casing 160A. A tip edge face 200 in the outer face of core 160B constituting the swirl chamber 177 has a gap to the opening 163 of the foregoing casing 160A, which constitutes a gas jet port 162. An edge part 201 in the inner face of core 160B has a gap to a liquid discharge port 162a, which constitutes a liquid discharge port 162b. The liquid discharge port 162b communicates with a liquid supply port 151b through the foregoing flow channel hole 168, gap 196 and flow channel 194.

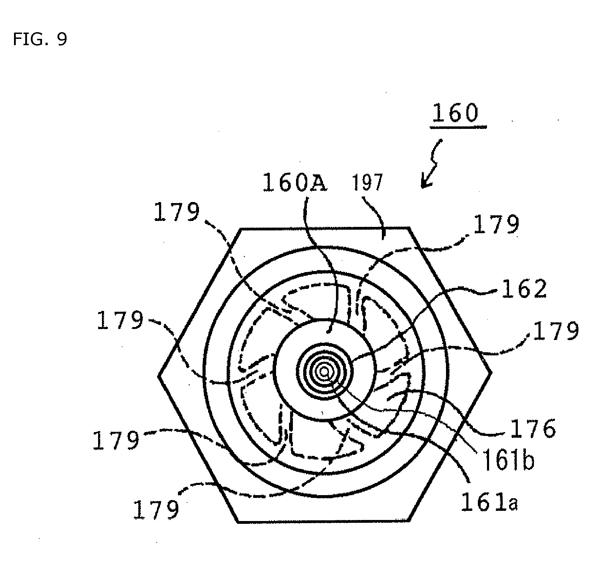

With reference to an elevation of two-fluid nozzle 160 shown in Fig. 9, a circular liquid discharge port 161a is disposed at the center, a liquid discharge port 161b in the periphery, and a circular gas jet port 162 further in the periphery are disposed. This gas jet port 162 communicates with a plurality of circling grooves 179 extending spirally that formed on the conical face of spiral forming body 176 being disposed inside the casing 160A.

A compressed gas supplied from the gas supply port 164 is passed through a space 170, and compressed in passing through a circling groove 179 with a small cross-sectional area formed in a spiral forming body 176, thereby to become a high-speed gas stream. This high-speed gas stream becomes a spiral circulating gas stream inside a swirl chamber 177, and discharged from a cylindrical gas jet port 162 narrowed down, thereby to form a high-speed swirling stream of gas forward from the two-fluid nozzle 160. This swirling stream is formed in a tapered conical form that the front position adjacent to the tip of casing 160A is a focal point.

A raw material liquid 112a fed from the first raw material tank 111a is passed through a raw material feeding pipe 121a and supplied to a solution sending tube 191 (liquid supply port 151a). The raw material liquid 112a supplied to the solution sending tube 191 is discharged from a liquid discharge port 161a. A raw material liquid 112b fed from the second raw material tank 111b is passed through a raw material feeding pipe 121b and supplied to a liquid supply port 151b. The raw material liquid 112b supplied to the liquid supply port 151b is discharged from a liquid discharge port 161b. These discharge flows are crushed into minute particles at the same time by a high-speed swirling stream of gas jetted from a gas jet port 162, and mixed forcibly being accompanied by rotation of swirling steam, thereby to be released forward from the two-fluid nozzle 160 as a group of misty particulates dispersed uniformly.

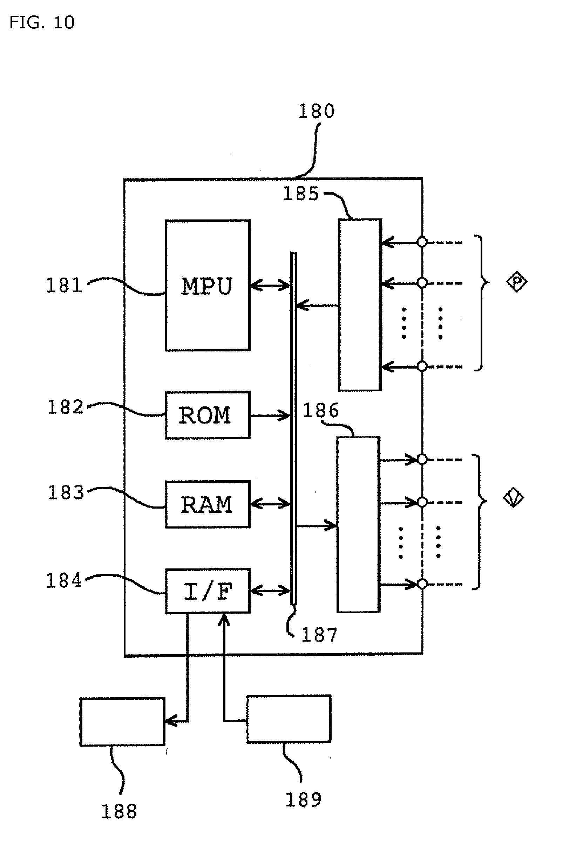

Production equipment 100 is controlled by a control device 180 shown in Fig. 10. The control device 180 houses a MPU 181, ROM 182, RAM 183, interface unit 184, A/D converter 185 and driving unit 186, and these are connected each other via a busline 187. In the ROM 182, a program that the MPU 181 runs is stored. The RAM 183 is used as a work area and the like when the MPU 181 runs the program. To an output port of interface unit 184, a display device 188 such as CRT is connected, and to an input port, an input device 189 such as keyboard is connected.

To the input of A/D converter 185, pressure sensors 135a, 135b and 138 of production equipment 100 are connected, and analog values of air pressures detected by these sensors are converted to digital values. Then, the values of air pressures converted to digital values are read by the MPU 181 via the busline 187.

The output of driving unit 186 is connected to electromagnetic driving valves 123a, 123b, 134a, 134b, 137 and 140 of production equipment 100. The driving unit 186 controls electric currents for the electromagnetic driving by instructions from the MPU 181, and switches ON/OF.

In operating the production equipment 100, an operator puts raw material liquids each in both raw material tanks 111a and 111b, and tightly seals the lids of both raw material tanks 111a and 111b. Thereafter, the operator orders a mixing start from the input device 189. Upon receiving this order, the MPU 181 sends an order to the driving unit 186 to open the electromagnetic valve 134, and also monitors the output of pressure sensors 135a and 135b via the A/D converter 185, and waits until a compressed gas from the compressor 133 is filled in the upper spaces of both raw material tanks 111a and 111b to reach a predetermined pressure. In this initial condition, other electromagnetic valves of production equipment 100 are closed. When the inside of each raw material tank is pressured up to a predetermined pressure and this is confirmed by pressure sensors 135a and 135b, the MPU 181 shuts down the electromagnetic valves 134a and 134b. Thereafter, the electromagnetic valve 137 is opened. Thereby, a compressed gas is supplied into a compressed gas reservoir 139.

Once the inner pressure of compressed gas reservoir 139 rises to a predetermined pressure, the MPU 181 judges that the condition for start of treatment was satisfied, and opens a pressure control valve 140. Now, a compressed gas is supplied to a gas supply port 152 of two-fluid nozzle 160 from the compressed gas reservoir 139, and a high-speed swirling stream of gas is jetted from the gas jet port 162 of the tip of two-fluid nozzle 160. Next, the MPU 181 opens electromagnetic variable throttles 123a and 123b so as to be a predetermined degree of opening. Now, the raw material liquids 112a and 112b are supplied to liquid supply ports 151a and 151b of two-fluid nozzle 160 through each raw material feeding pipe 121a and 121b, and discharged from liquid discharge ports 161a and 161b of the tip of two-fluid nozzle 160. The raw material liquids 112a and 112b discharged from the two-fluid nozzle 160 are crushed into minute particles by the high-speed swirling stream of air that has already been formed in a discharge direction, being accompanied by the swirling stream, the raw material liquids 112a and 112b become a uniform state, and are released into a production container 125.

As the foregoing treatment proceeds, since fluid levels of raw material liquids 112a and 112b of raw material tanks 111a and 111b respectively are lowered, volumes of upper spaces of both raw material tanks 111a and 111b increase, and pressures are lowered being accompanied thereby. The pressures are always detected by the pressure sensors 135a and 135b, and the values are sent to the MPU 181. The MPU 181 always monitors the detected value by the pressure sensors 135a and 135b, and when the value is less than a suitable value, switches the electromagnetic valves 134a and 134b to an open condition for a suitable time, thereby to keep the inner pressure of raw material tanks 111a and 111b in a predetermined suitable value. Similarly, the pressure of the compressed gas inside the compressed gas reservoir 139 is kept in a suitable value by controlling the electromagnetic valve 137 by the MPU 181.

According to the treatment equipment 100 of the present embodiment, when a liquid that a membrane material is dissolved or dispersed as a raw material liquid 112a, and an aqueous medium as a raw material liquid 112b are used, by the operation explained above, liposome dispersion is produced as a dispersion 124 and, accommodated and recovered in a recovery container 125. This dispersion 124 is in a uniformly dispersed state as liposome dispersion, and the dispersion state can be maintained for a long time, because while a liquid that a membrane material is dissolved or dispersed and an aqueous medium are discharged from liquid discharge ports 161a and 161b, respectively, they are crushed by a high-speed swirling stream jetted from the liquid jet port 162 disposed in the periphery of the liquid discharge port 161b, and the flow right after crush is reaggregated by crashing against a flow-blocking body 190 for the production.

In this case, in the conventional equipment that raw material liquids 112a and 112b are previously mixed and discharged from one discharge port, there is a problem that the two liquids separate in a flow channel in a traveling direction because of viscosity difference between the raw material liquids 112a and 112b, or the like, but in the treatment equipment 100 of the present embodiment, raw material liquids 112a and 112b are not mixed before discharge, thus such problem does not occur, uniformity when dispersed in a minute particle state is further improved, and uniformity of raw material liquids 112a and 112b as dispersion can be further enhanced.

### [Examples]

Hereinafter, the present invention will be detailed in reference to Examples, but the present invention is not limited to the present Examples. Additionally, as a two-fluid nozzle used for treatment in the present Examples, a two-fluid nozzle (one discharge port) described in Japanese Unexamined Patent Publication Nos. 2005-295856 and 2003-62493 was used.

### (Measurement of particle diameter of liposome)

### (1) Experimental technique

### 1) Experiment using monoolein

A n-decane solution (monoolein concentration: 5mg/mL) of monoolein (C₂₁H₄O₄, MW=356.55) was prepared. A mixture that the solution prepared and a 10 mM NaCl aqueous solution were mixed for the volume ratio to be 1:1 was treated by a mixer (a type spraying it from a two-fluid nozzle and crashing against a baffle board 190 similar to treatment equipment 100 for aggregation). This treatment was repeated one to three times, and the dispersion after each treatment was diluted with n-decane by 100 times.

### 2) Experiment using phosphatidyl choline

A n-decane solution (phosphatidyl choline concentration: 1.25 mg/mL) of phosphatidyl choline (derived from egg yolk) was prepared. A mixture that the solution prepared and a 10 mM NaCl aqueous solution were mixed for the volume ratio to be 1:1 was treated by a mixer (a type spraying it from a two-fluid nozzle and crashing against a baffle board 190 similar to treatment equipment 100 for aggregation). This treatment was repeated one to three times, and the dispersion after each treatment was diluted with a 10 mM NaCl aqueous solution by 100 times.

### (2) Evaluation method

Each sample was analyzed for a particle size distribution by a cumulant method using a light-scattering photometer (dynamic light-scattering photometer, DLS-7000 (manufactured by Otsuka Electronics Co., Ltd.).

### (3) (Evaluation result)

Table 1 summarized the average of particle diameter (µm) and ratio (%) in the sample treated by a mixer at a sample pressure gauge of 0.2 MPa and air jet pressure of 0.5 MPa. Table 2 showed the experimental result conducted in another condition (the same condition as in Table 1 except that it was changed to monoolein concentration: 50 mg/L, phosphatidyl choline concentration: 25 mg/mL). Additionally, in Table 1 and Table 2, M means monoolein and P means phosphatidyl choline. It is known from the result that liposome with a small particle diameter and narrow distribution was obtained in each sample. Further, it is known that the control of particle diameter is possible and particle diameter can be made small by conducting the treatment repeatedly.

**[Table 1]**

| Sample | Peak 1 | Peak 2 | Peak 3 |
|---|---|---|---|
| M two-time treatment | 1.248 (93.9%) | 0.463 (6.1%) | - |
| M three-time treatment | 1.067 (89.9%) | - | - |
| P two-time treatment | 1.371 (8.5%) | 0.253 (91.5%) | - |
| P three-time treatment | 0.985 (99.6%) | - | - |

**[Table 2]**

| Sample | Peak 1 | Peak 2 | Peak 3 |
|---|---|---|---|
| M one-time treatment | 3.092 | - | - |
| M one-time treatment | 3.265 | - | - |
| M two-time treatment | 4.025 | 1.118 | - |
| P one-time treatment | 12.086 | 1.848 | 0.820 |
| P two-time treatment | 6.351 | 1.132 | - |

### INDUSTRIAL APPLICABILITY

The production method of liposome dispersion and production equipment according to the present invention can prepare liposome dispersion at short times and in large amounts, and it is expected to be applied as a production method of liposome dispersion in an industrial scale. Further, it can use delicate materials such as proteins and polymer compounds.

## Claims

1. A production method of liposome dispersion, in a process for forming liposome by dispersing a membrane material in an aqueous medium,
wherein the membrane material is dispersed in the aqueous medium by spray mixing.

2. The production method of claim 1, wherein while discharging a liquid that the membrane material is suspended in the aqueous medium, the discharge flow is crushed by a gas stream to produce minute droplets, then, the droplets are aggregated.

3. The production method of claim 1, wherein while discharging a liquid that the membrane material is dissolved or dispersed in a solvent and the aqueous medium, the discharge flow is crushed by a gas stream to produce minute droplets, then the droplets are aggregated.

4. Production equipment of liposome dispersion, which is equipment for producing liposome dispersion, comprising; a nozzle equipped with:
a first discharge port for discharging a liquid that a membrane material is dissolved or dispersed,
a second discharge port equipped adjacent to the first discharge port for discharging an aqueous medium, and
a gas jet port jetting a gas for collectively crushing discharge flows from the first discharge port and the second discharge port to produce minute droplets;
a first solution-sending means for supplying a liquid that the membrane material is dissolved or dispersed to the nozzle;
a second solution-sending means for supplying the aqueous medium to the nozzle; and
a gas supply means for supplying the gas to the nozzle.

5. The production equipment of claim 4, wherein the first discharge port is circular,
the second discharge port is formed in an annular shape surrounding the first discharge port, and
the gas jet port is formed in the periphery of the second discharge port.

6. The production equipment of claim 4 or 5, wherein a flow-blocking body is provided oppositely to the first discharge port and the second discharge port, and constituted so that a flow of minute droplets produced by crushing the liquid that the membrane material is dissolved or dispersed and the aqueous medium by the gas stream is aggregated by crashing against the flow-blocking body.
